Europäisches Patentamt
European Patent Office
Office européen des brevets

(19) (11) Veröffentlichungsnummer: **0 173 284**
**A1**

(12)

# EUROPÄISCHE PATENTANMELDUNG

(21) Anmeldenummer: **85110695.5**

(22) Anmeldetag: **27.08.85**

(51) Int. Cl.⁴: **C 07 D 207/40**, A 01 N 43/36

(30) Priorität: **30.08.84 DE 3431873**

(43) Veröffentlichungstag der Anmeldung: **05.03.86**
**Patentblatt 86/10**

(84) Benannte Vertragsstaaten: **BE DE FR GB IT NL**

(71) Anmelder: **Consortium für elektrochemische Industrie GmbH, Zielstattstrasse 20, D-8000 München 70 (DE)**

(72) Erfinder: **Häberle, Norman, Dr. Dipl.-Chem., Willibaldstrasse 51 a, D-8000 München 21 (DE)**
Erfinder: **Reutter, Anneliese, Dipl.-Ing. (FH), Hirschenweg 10, D-8011 Eglharting (DE)**
Erfinder: **Kinzel, Peter, Dipl.-Ing. (FH), Mareisring 30, D-8151 Feldkirchen (DE)**

(54) **Substituierte 1-Phenyl-3-methyl-pyrrolidindione, ihre Herstellung und Verwendung als Fungizide.**

(57) Die Erfindung betrifft Verbindungen der allgemeinen Formel

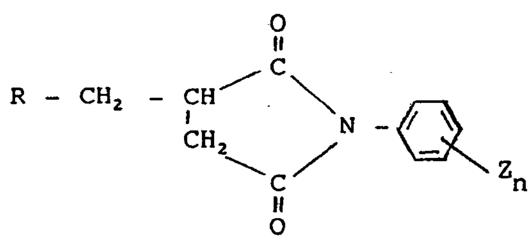

wobei

R F, Cl, Br, J, CN, SCN und Methansulfonyl bedeutet,

Z für F, Cl, Br, J, $NO_2$, CN, SCN, Sulfamoyl, Phenoxy, Alkyl mit 1 bis 3 Kohlenstoffatomen, Halogenalkyl mit 1 bis 3 Kohlenstoffatomen und 1 bis 7 Halogenatomen aus der Gruppe F, Cl, Br; Alkoxy mit 1 bis 3 Kohlenstoffatomen, Halogenalkoxy mit 1–3 Kohlenstoff- und 1–4 Halogenatomen aus der Gruppe F, Cl, Br; Allyloxy und Ethoxycarbonyl steht,

n 0, oder, mit der Massgabe, dass n 1, 2, 3 oder 4 ist, für folgenden Substitutionstyp steht: 2-, 4-; 2.3-, 2.4-, 2.5-, 2.6-, 3.4-; 2.3.4-, 2.3.5-, 2.3.6-, 2.4.5-, 2.4.6-, 3.4.5-; 2.3.4.5-, 2.4.5.6-, 2.3.5.6-; die erfindungsgemässen Verbindungen weisen fungizide Wirksamkeit auf.

EP 0 173 284 A1

0 173 284

Consortium
für elektrochemische Industrie
GmbH

München, den 23.8.1984
PAT/Dr.Ra/we

Co 8409

Substituierte 1-Phenyl-3-methyl-pyrrolidindione, ihre Herstellung und Verwendung als Fungizide

Die Erfindung betrifft neue 1-Phenyl-3-methyl-pyrrolidin-2.5-dione, die am Methylrest Substituenten aufweisen.

1-Phenyl-pyrrolidin-2.5-dione und ihre fungizide Wirksamkeit, insbesondere gegen Botrytis cinerea, sind bereits bekannt. Es wurde nun in letzter Zeit mehrfach über Resistenzerscheinungen bei Botrytis cinerea berichtet, die insbesondere auch bei den bisher als besonders wirksamen 1-(3.5-Dichlorphenyl)-pyrrolidindionen festgestellt wurden. Hierzu sei auf M. Grindle, Pestic. Sci. 12, 305, (1982) verwiesen.

Weiterhin sind gemäß Jap. Kokai 75/107.137 Chlormethylmaleinsäureanilide bekannt. Diese Verbindungen sind nachteiligerweise chemisch schwer zugänglich, unter Anwendungsbedingungen unbeständig und weisen zudem ein lediglich schmales Wirkungsspektrum auf.

Aufgabe der Erfindung war es, neue fungizide Wirkstoffe aufzufinden. Weiterhin war es Aufgabe der Erfindung, solche Wirkstoffe aufzufinden, die auch unter Lichteinfluß stabil sind, chemisch leicht zugänglich sind und die auch solche Schadpilze sicher bekämpfen, die gegen ausschließlich meta- bzw. meta/meta substituierte Dicarboxanilide bereits Resistenzerscheinungen zeigen.

Gegenstand der Erfindung sind Verbindungen der allgemeinen Formel

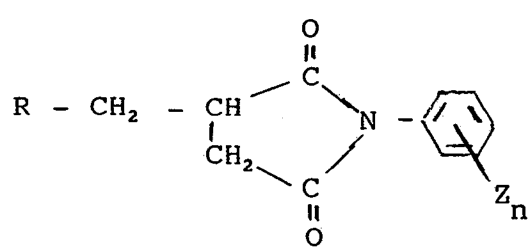

wobei

R F, Cl, Br, J, CN, SCN und Methansulfonyl bedeutet,

Z für F, Cl, Br, J, $NO_2$, CN, SCN, Sulfamoyl, Phenoxy, Alkyl mit 1 bis 3 Kohlenstoffatomen, Halogenalkyl mit 1 bis 3 Kohlenstoffatomen und 1 bis 7 Halogenatomen aus der Gruppe F, Cl, Br; Alkoxy mit 1 bis 3 Kohlenstoffatomen, Halogenalkoxy mit 1-3 Kohlenstoff- und 1-4 Halogenatomen aus der Gruppe F, Cl, Br; Allyloxy und Ethoxycarbonyl steht

n 0, oder, mit der Maßgabe, daß n 1, 2, 3 oder 4 ist, für folgenden Substitutionstyp steht: 2-, 4-; 2.3-, 2.4-, 2.5-, 2.6-, 3.4-; 2.3.4-, 2.3.5-, 2.3.6-, 2.4.5-, 2.4.6-, 3.4.5-; 2.3.4.5-, 2.4.5.6-, 2.3.5.6-;

Beispiele für Alkylgruppen mit der Bedeutung Z sind Methyl-, Ethyl-, n-Propyl-, i-Propyl-. Beispiele für Halogenalkylgruppen mit der Bedeutung Z sind Trifluormethyl, Pentafluorethyl, Heptafluorpropyl, Chlormethyl, Dichlormethyl, Trichlormethyl und Brommethyl. Beispiele für Alkoxygruppen mit der Bedeutung Z sind Methoxy-, Ethoxy- und Propoxy-. Beispiele für Halogenalkoxygruppen mit der Bedeutung Z sind insbesondere die Difluormethoxy- und die Tetrafluorethoxygruppe.

Spezielle Beispiele für erfindungsgemäße Verbindungen sind:

1) 3-Chlormethyl-1-phenyl-pyrrolidin-2.5-dion
2) 3-Chlormethyl-1-(4-fluorphenyl)-pyrrolidin-2.5-dion
3) 3-Chlormethyl-1-(2-chlorphenyl)-pyrrolidin-2.5-dion
4) 3-Chlormethyl-1-(4-chlorphenyl)-pyrrolidin-2.5-dion
5) 3-Chlormethyl-1-(2-bromphenyl)-pyrrolidin-2.5-dion
6) 3-Chlormethyl-1-(4-bromphenyl)-pyrrolidin-2.5-dion
7) 3-Chlormethyl-1-(4-jodphenyl)-pyrrolidin-2.5-dion
8) 3-Chlormethyl-1-(2.4-difluorphenyl)-pyrrolidin-2.5-dion
9) 3-Chlormethyl-1-(2.6-difluorphenyl)-pyrrolidin-2.5-dion
10) 3-Chlormethyl-1-(2.3-dichlorphenyl)-pyrrolidin-2.5-dion
11) 3-Chlormethyl-1-(2.4-dichlorphenyl)-pyrrolidin-2.5-dion
12) 3-Chlormethyl-1-(2.5-dichlorphenyl)-pyrrolidin-2.5-dion

13) 3-Chlormethyl-1-(4-chlor-2-fluor-phenyl)-pyrrolidin-2.5-dion
14) 3-Chlormethyl-1-(2-chlor-4-fluor-phenyl)-pyrrolidin-2.5-dion
15) 3-Chlormethyl-1-(4-methylphenyl)-pyrrolidin-2.5-dion
16) 3-Chlormethyl-1-(4-propylphenyl)-pyrrolidin-2.5-dion
17) 3-Chlormethyl-1-(2.6-dimethylphenyl)-pyrrolidin-2.5-dion
18) 3-Chlormethyl-1-(2.6-diethylphenyl)-pyrrolidin-2.5-dion
19) 3-Chlormethyl-1-(5-fluor-2-methyl-phenyl)-pyrrolidin-2.5-dion
20) 3-Chlormethyl-1-(2-chlor-6-methyl-phenyl)-pyrrolidin-2.5-dion
21) 3-Chlormethyl-1-(2-trifluormethyl-phenyl)-pyrrolidin-2.5-dion
22) 3-Fluormethyl-1-(4-bromphenyl)-pyrrolidin-2.5-dion
23) 3-Fluormethyl-1-(4-jodphenyl)-pyrrolidin-2.5-dion
24) 3-Fluormethyl-1-(2.4-difluorphenyl)-pyrrolidin-2.5-dion
25) 3-Fluormethyl-1-(2.6-difluorphenyl)-pyrrolidin-2.5-dion
26) 3-Fluormethyl-1-(2.3-difluorphenyl)-pyrrolidin-2.5-dion
27) 3-Fluormethyl-1-(2.4-dichlorphenyl)-pyrrolidin-2.5-dion
28) 3-Fluormethyl-1-(2.5-dichlorphenyl)-pyrrolidin-2.5-dion
29) 3-Fluormethyl-1-(4-chlor-2-fluor-phenyl)-pyrrolidin-2.5-dion
30) 3-Fluormethyl-1-(2-chlor-4-fluor-phenyl)-pyrrolidin-2.5-dion
31) 3-Fluormethyl-1-(4-methylphenyl)-pyrrolidin-2.5-dion
32) 3-Fluormethyl-1-(4-propylphenyl)-pyrrolidin-2.5-dion
33) 3-Fluormethyl-1-(2.6-dimethylphenyl)-pyrrolidin-2.5-dion
34) 3-Fluormethyl-1-(2.6-diethylphenyl)-pyrrolidin-2.5-dion
35) 3-Fluormethyl-1-(5-fluor-2-methyl-phenyl)-pyrrolidin-2.5-dion
36) 3-Fluormethyl-1-(2-chlor-6-methyl-phenyl)-pyrrolidin-2.5-dion
37) 3-Fluormethyl-1-(2-trifluormethylphenyl)-pyrrolidin-2.5-dion
38) 3-Chlormethyl-1-(4-trifluormethylphenyl)-pyrrolidin-2.5-dion
39) 3-Chlormethyl-1-(4-chlor-2-trifluormethyl-phenyl)-pyrrolidin-2.5-dion
40) 3-Chlormethyl-1-(4-methoxyphenyl)-pyrrolidin-2.5-dion
41) 3-Chlormethyl-1-(4-allyloxyphenyl)-pyrrolidin-2.5-dion
42) 3-Chlormethyl-1-(4-difluormethoxyphenyl)-pyrrolidin-2.5-dion
43) 3-Chlormethyl-1-[2-(1.1.2.2-tetrafluorethoxy)-phenyl]-pyrrolidin-2.5-dion
44) 3-Chlormethyl-1-[4-(1.1.2.2-tetrafluorethoxy)-phenyl]-pyrrolidin-2.5-dion
45) 3-Chlormethyl-1-[4-(2.3-dibrom)-propoxy-phenyl]-pyrrolidin-2.5-dion

46) 3-Chlormethyl-1-(4-nitrophenyl)-pyrrolidin-2.5-dion

47) 3-Chlormethyl-1-(4-fluor-3-nitrophenyl)-pyrrolidin-2.5-dion

48) 3-Chlormethyl-1-(4-phenoxyphenyl)-pyrrolidin-2.5-dion

49) 3-Chlormethyl-1-(4-ethoxycarbonylphenyl)-pyrrolidin-2.5-dion

50) 3-Chlormethyl-1-(4-cyanophenyl)-pyrrolidin-2.5-dion

51) 3-Chlormethyl-1-(4-thiocyanatophenyl)-pyrrolidin-2.5-dion

52) 3-Chlormethyl-1-(4-sulfamoylphenyl)-pyrrolidin-2.5-dion

53) 3-Chlormethyl-1-(2.4-dimethylphenyl)-pyrrolidin-2.5-dion

54) 3-Chlormethyl-1-(3-fluor-2-methyl-phenyl)-pyrrolidin-2.5-dion

55) 3-Chlormethyl-1-(4-fluor-2-methyl-phenyl)-pyrrolidin-2.5-dion

56) 3-Chlormethyl-1-(4-fluor-3-methyl-phenyl)-pyrrolidin-2.5-dion

57) 3-Chlormethyl-1-(4-chlor-2-methyl-phenyl)-pyrrolidin-2.5-dion

58) 3-Chlormethyl-1-(4-brom-2-methyl-phenyl)-pyrrolidin-2.5-dion

59) 3-Chlormethyl-1-(4-brom-3-methyl-phenyl)-pyrrolidin-2.5-dion

60) 3-Chlormethyl-1-(2-methyl-4-nitro-phenyl)-pyrrolidin-2.5-dion

61) 3-Chlormethyl-1-(2-methyl-6-nitro-phenyl)-pyrrolidin-2.5-dion

62) 3-Chlormethyl-1-(2.4.6-trimethylphenyl)-pyrrolidin-2.5-dion

63) 3-Chlormethyl-1-(4-brom-2.6-dimethyl-phenyl)-pyrrolidin-2.5-dion

64) 3-Chlormethyl-1-(2.4.6-trifluorphenyl)-pyrrolidin-2.5-dion

65) 3-Chlormethyl-1-(2.3.5.6-tetrafluorphenyl)-pyrrolidin-2.5-dion

66) 3-Chlormethyl-1-(2.4.5-trimethylphenyl)-pyrrolidin-2.5-dion dion

67) 3-Fluormethyl-1-(3-fluor-2-methyl-phenyl)-pyrrolidin-2.5-dion

68) 3-Fluormethyl-1-(4-chlor-2-methyl-phenyl)-pyrrolidin-2.5-dion

69) 3-Fluormethyl-1-(4-brom-2-methyl-phenyl)-pyrrolidin-2.5-dion
70) 3-Fluormethyl-1-(4-brom-3-methyl-phenyl)-pyrrolidin-2.5-dion
71) 3-Fluormethyl-1-(2.4-dimethylphenyl)-pyrrolidin-2.5-dion
72) 3-Fluormethyl-1-(2-methyl-6-nitro-phenyl)-pyrrolidin-2.5-dion
73) 3-Brommethyl-1-(4-fluorphenyl)-pyrrolidin-2.5-dion
74) 3-Brommethyl-1-(4-chlorphenyl)-pyrrolidin-2.5-dion
75) 3-Brommethyl-1-(2-fluorphenyl)-pyrrolidin-2.5-dion
76) 3-Brommethyl-1-(4-methylphenyl)-pyrrolidin-2.5-dion
77) 3-Brommethyl-1-(4-bromphenyl)-pyrrolidin-2.5-dion
78) 3-Brommethyl-1-(4-jodphenyl)-pyrrolidin-2.5-dion
79) 3-Brommethyl-1-(5-fluor-2-methyl-phenyl)-pyrrolidin-2.5-dion
80) 3-Brommethyl-1-(2-chlor-5-trifluormethyl-phenyl)-pyrrolidin-2.5-dion
81) 3-Jodmethyl-1-(4-methylphenyl)-pyrrolidin-2.5-dion
82) 3-Jodmethyl-1-phenyl-pyrrolidin-2.5-dion
83) 3-Jodmethyl-1-(4-fluorphenyl)-pyrrolidin-2.5-dion
84) 3-Jodmethyl-1-(4-chlorphenyl)-pyrrolidin-2.5-dion
85) 3-Jodmethyl-1-(2.4-difluorphenyl)-pyrrolidin-2.5-dion
86) 3-Jodmethyl-1-(2.6-difluorphenyl)-pyrrolidin-2.5-dion
87) 3-Jodmethyl-1-(2.3-dichlorphenyl)-pyrrolidin-2.5-dion
88) 3-Jodmethyl-1-(2.4-dichlorphenyl)-pyrrolidin-2.5-dion
89) 3-Jodmethyl-1-(2-trifluormethylphenyl)-pyrrolidin-2.5-dion
90) 3-Jodmethyl-1-(4-trifluormethylphenyl)-pyrrolidin-2.5-dion
91) 3-Jodmethyl-1-(4-allyloxyphenyl)-pyrrolidin-2.5-dion
92) 3-Jodmethyl-1-(4-difluormethoxyphenyl)-pyrrolidin-2.5-dion
93) 3-Jodmethyl-1-[2-(1.1.2.2-tetrafluorethoxy)-phenyl]-pyrrolidin-2.5-dion
94) 3-Jodmethyl-1-[4-(1.1.2.2-tetrafluorethoxy)-phenyl]-pyrrolidin-2.5-dion
95) 3-Jodmethyl-1-(4-bromphenyl)-pyrrolidin-2.5-dion
96) 3-Jodmethyl-1-(4-jodphenyl)-pyrrolidin-2.5-dion
97) 3-Jodmethyl-1-(4-cyanophenyl)-pyrrolidin-2.5-dion
98) 3-Jodmethyl-1-(4-fluor-2-methyl-phenyl)-pyrrolidin-2.5-dion
99) 3-Jodmethyl-1-(4-chlor-2-methyl-phenyl)-pyrrolidin-2.5-dion

100) 3-Jodmethyl-1-(2.6-dimethyl-phenyl)-pyrrolidin-2.5-dion

101) 3-Jodmethyl-1-(2.4.5-trimethylphenyl)-pyrrolidin-2.5-dion

102) 3-Jodmethyl-1-(2.4.6-trimethylphenyl)-pyrrolidin-2.5-dion

103) 3-Jodmethyl-1-(2-chlor-4-sulfamoyl-phenyl)-pyrrolidin-2.5-dion

104) 3-Jodmethyl-1-(2-fluor-5-nitro-phenyl)-pyrrolidin-2.5-dion

105) 3-Jodmethyl-1-(2-methoxy-5-methyl-phenyl)-pyrrolidin-2.5-dion

106) 3-Jodmethyl-1-(2.4.5-trichlorphenyl)-pyrrolidin-2.5-dion

107) 3-Jodmethyl-1-(5-chlor-2-methyl-phenyl)-pyrrolidin-2.5-dion

108) 3-Jodmethyl-1-(2-chlor-5-methyl-phenyl)-pyrrolidin-2.5-dion

109) 3-Jodmethyl-1-(2-chlor-5-trifluormethyl-phenyl)-pyrrolidin-2.5-dion

110) 3-Jodmethyl-1-(4-chlor-3-fluor-5-methyl-phenyl)-pyrrolidin-2.5-dion

111) 3-Cyanomethyl-1-(4-fluorphenyl)-pyrrolidin-2.5-dion

112) 3-Cyanomethyl-1-(2-fluor-5-methyl-phenyl)-pyrrolidin-2.5-dion

113) 3-Cyanomethyl-1-(4-chlor-3-fluor-5-methyl-phenyl)-pyrrolidin-2.5-dion

114) 3-Cyanomethyl-1-(2-chlor-5-trifluormethyl-phenyl)-pyrrolidin-2.5-dion

115) 3-Cyanomethyl-1-(2-chlor-5-methyl-phenyl)-pyrrolidin-2.5-dion

116) 3-Cyanomethyl-1-(5-chlor-2-methyl-phenyl)-pyrrolidin-2.5-dion

117) 3-Cyanomethyl-1-(2.4.5-trichlorphenyl)-pyrrolidin-2.5-dion

118) 3-Cyanomethyl-1-(2-methoxy-5-methyl-phenyl)-pyrrolidin-2.5-dion

119) 3-Cyanomethyl-1-(2-fluor-5-nitro-phenyl)-pyrrolidin-2.5-dion

120) 3-Cyanomethyl-1-(2-chlor-4-sulfamoyl-phenyl)-pyrrolidin-2.5-dion

121) 3-Cyanomethyl-1-(2.4.6-trimethylphenyl)-pyrrolidin-2.5-dion

122) 3-Cyanomethyl-1-(2.4.5-trimethylphenyl)-pyrrolidin-2.5-dion

123) 3-Thiocyanatomethyl-1-(4-fluorphenyl)-pyrrolidin-2.5-dion

124) 3-Thiocyanatomethyl-1-(2-fluor-5-methyl-phenyl)-pyrrolidin-2.5-dion

125) 3-Thiocyanatomethyl-1-(4-chlor-3-fluor-5-methyl-phenyl)-pyrrolidin-2.5-dion

126) 3-Thiocyanatomethyl-1-(2-chlor-5-trifluormethyl-phenyl)-pyrrolidin-2.5-dion

127) 3-Thiocyanatomethyl-1-(2-chlor-5-methyl-phenyl)-pyrrolidin-2.5-dion

128) 3-Thiocyanatomethyl-1-(5-chlor-2-methyl-phenyl)-pyrrolidin-2.5-dion

129) 3-Thiocyanatomethyl-1-(2.4.5-trichlorphenyl)-pyrrolidin-2.5-dion

130) 3-Thiocyanatomethyl-1-(2-methoxy-5-methyl-phenyl)-pyrrolidin-2.5-dion

131) 3-Thiocyanatomethyl-1-(2-fluor-5-nitro-phenyl)-pyrrolidin-2.5-dion

132) 3-Thiocyanatomethyl-1-(2-chlor-4-sulfamoyl-phenyl)-pyrrolidin-2.5-dion

133) 3-Thiocyanatomethyl-1-(2.4.6-trimethylphenyl)-pyrrolidin-2.5-dion

134) 3-Thiocyanatomethyl-1-(2.4.5-trimethylphenyl)-pyrrolidin-2.5-dion

135) 3-Methansulfonylmethyl-1-(4-fluorphenyl)-pyrrolidin-2.5-dion

136) 3-Methansulfonylmethyl-1-(2-fluor-5-methyl-phenyl)-pyrrolidin-2.5-dion

137) 3-Methansulfonylmethyl-1-(4-chlor-3-fluor-5-methyl-phenyl)-pyrrolidin-2.5-dion

138) 3-Methansulfonylmethyl-1-(2-chlor-5-trifluormethyl-phenyl)-pyrrolidin-2.5-dion

139) 3-Methansulfonylmethyl-1-(2-chlor-5-methyl-phenyl)-pyrrolidin-2.5-dion

140) 3-Methansulfonylmethyl-1-(5-chlor-2-methyl-phenyl)-pyrrolidin-2.5-dion

141) 3-Methansulfonylmethyl-1-(2.4.5-trichlorphenyl)-pyrrolidin-2.5-dion

142) 3-Methansulfonylmethyl-1-(2-methoxy-5-methyl-phenyl)-pyrrolidin-2.5-dion

143) 3-Methansulfonylmethyl-1-(2-fluor-5-nitro-phenyl)-pyrrolidin-2.5-dion

144) 3-Methansulfonylmethyl-1-(2-chlor-4-sulfamoyl-phenyl)-pyrrolidin-2.5-dion

145) 3-Methansulfonylmethyl-1-(2.4.6-trimethylphenyl)-pyrrolidin-2.5-dion

146) 3-Methansulfonylmethyl-1-(2.4.5-trimethylphenyl)-pyrrolidin-2.5-dion

147) 3-Fluormethyl-1-(4-fluorphenyl)-pyrrolidin-2.5-dion

148) 3-Fluormethyl-1-(2-fluor-5-methyl-phenyl)-pyrrolidin-2.5-dion

149) 3-Fluormethyl-1-(4-chlor-3-fluor-5-methyl-phenyl)-pyrrolidin-2.5-dion

150) 3-Fluormethyl-1-(2-chlor-5-trifluormethyl-phenyl)-pyrrolidin-2.5-dion

151) 3-Fluormethyl-1-(2-chlor-5-methyl-phenyl)-pyrrolidin-2.5-dion

152) 3-Fluormethyl-1-(5-chlor-2-methyl-phenyl)-pyrrolidin-2.5-dion

153) 3-Fluormethyl-1-(2.4.5-trichlorphenyl)-pyrrolidin-2.5-dion

154) 3-Fluormethyl-1-(2-methoxy-5-methyl-phenyl)-pyrrolidin-2.5-dion

155) 3-Fluormethyl-1-(2-fluor-5-nitro-phenyl)-pyrrolidin-2.5-dion

156) 3-Fluormethyl-1-(2-chlor-4-sulfamoyl-phenyl)-pyrrolidin-2.5-dion

157) 3-Chlormethyl-1-(2,4-dibromphenyl)-pyrrolidin-2.5-dion

158) 3-Chlormethyl-1-(2,6-dichlorphenyl)-pyrrolidin-2.5-dion

159) 2-Brommethyl-1-(5-brom-2-methylphenyl)-pyrrolidin-2.5-dion

160) 3-Brommethyl-1-(4-chlor-2-fluor-5-methylphenyl)-pyrrolidin-2.5-dion

161) 3-Brommethyl-1-(2-chlor-5-methylphenyl)-pyrrolidin-2.5-dion

162) 3-Brommethyl-1-(2,5-dichlor-4-methylphenyl)-pyrrolidin-2.5-dion

163) 3-Brommethyl-1-(5-chlor-2-methylphenyl)-pyrrolidin-2.5-dion

164) 3-Brommethyl-1-(2,5-dichlorphenyl)-pyrrolidin-2.5-dion

165) 3-Brommethyl-1-(2,5-dichlor-4-methylphenyl)-pyrrolidin-2.5-dion

166) 3-Brommethyl-1-(2,3,4-trichlorphenyl)-pyrrolidin-2.5-dion

167) 3-Brommethyl-1-(2,4,5-trichlorphenyl)-pyrrolidin-2.5-dion

168) 3-Brommethyl-1-(4-cyanophenyl)-pyrrolidin-2.5-dion

169) 3-Brommethyl-1-(2,5-difluorphenyl)-pyrrolidin-2.5-dion

170) 3-Brommethyl-1-(2-fluor -5-methylphenyl)-pyrrolidin-2.5-dion

171) 3-Brommethyl-1-(2,4-difluor-5-methylphenyl)-pyrrolidin-2.5-dion

172) 3-Brommethyl-1-(2-fluor-5-nitrophenyl)-pyrrolidin-2.5-dion

173) 3-Brommethyl-1-(2-trifluormethylphenyl)-pyrrolidin-2.5-dion

174) 3-Brommethyl-1-(4-trifluormethylphenyl)-pyrrolidin-2.5-dion

175) 3-Brommethyl-1-(2-methoxy-5-methylphenyl)-pyrrolidin-2.5-dion

176) 3-Brommethyl-1-(3-methoxy-4-methylphenyl)-pyrrolidin-2.5-dion

177) 3-Brommethyl-1-(2-methoxy-5-methyl-4-nitrophenyl)-pyrrolidin-2.5-dion

178) 3-Brommethyl-1-(2,3-dimethyl-4-nitrophenyl)-pyrrolidin-2.5-dion

179) 3-Brommethyl-1-(4,5-dimethyl-2-nitrophenyl)-pyrrolidin-2.5-dion

180) 3-Jodmethyl-1-(5-chlor-2-methylphenyl)-pyrrolidin-2.5-dion

181) 3-Jodmethyl-1-(2-methyl-5-nitrophenyl)-pyrrolidin-2.5-dion

182) 3-Brommethyl-1-(5-chlor-2-methoxyphenyl)-pyrrolidin-2.5-dion

Durch das Asymmetriezentrum in 3-Stellung der erfindungsgemäßen 2,5-Pyrrolidindione wird bedingt, daß Enantiomere entstehen, die jeweils einzeln und als racemisches Gemisch beansprucht werden.

Die erfindungsgemäßen Verbindungen sind durch Umsetzen entsprechend substituierter Bernsteinsäuren, deren Säurechloriden bzw. deren Anhydriden mit den entsprechend substituierten Anilinen zugänglich.

Ein bevorzugtes Verfahren zur Herstellung der erfindungsgemäßen Verbindungen ist dadurch gekennzeichnet, daß Bernsteinsäuren, deren Säurechloride bzw. deren Anhydride, die in der 2-Stellung durch einen

$R - CH_2$-Rest

substituiert sind, wobei
R F, Cl, Br, J, CN, SCN und Methansulfonyl bedeutet,
mit Anilinen der Formel

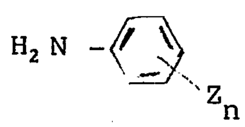

wobei

Z für F, Cl, Br, J, $NO_2$, CN, SCN, Sulfamoyl, Phenoxy, Alkyl mit 1 bis 3 Kohlenstoffatomen, Halogenalkyl mit 1 bis 3 Kohlenstoffatomen und 1 bis 7 Halogenatomen aus der Gruppe F, Cl, Br; Alkoxy mit 1 bis 3 Kohlenstoffatomen, Halogenalkoxy mit 1-3 Kohlenstoff- und 1-4 Halogenatomen aus der Gruppe F, Cl, Br; Allyloxy und Ethoxycarbonyl steht,

n 0 oder, mit der Maßgabe, daß n 1, 2, 3 oder 4 ist, für folgenden Substitutionstyp steht: 2-, 4-; 2.3-, 2.4-, 2.5-, 2.6-, 3.4-; 2.3.4-, 2.3.5-, 2.3.6-, 2.4.5-, 2.4.6-, 3.4.5-; 2.3.4.5-, 2.4.5.6-, 2.3.5.6-;

umgesetzt werden.

Bei den vorstehend mit n bezeichneten Substitutionstypen steht die Aminfunktion des Anilins stets in 1-Stellung des Phenylkerns.

In einer besonders vorteilhaften Verfahrensvariante wird die Umsetzung der Bernsteinsäurederivate mit den entsprechenden Anilinen in Gegenwart eines inerten Lösungsmittels, wie beispielsweise Xylol, ausgeführt und das sich bildende Reaktionswasser durch azeotrope Destillation entfernt. Tertiäre Amine, wie Tributylamin oder Pyridin u.a. beschleunigen dabei die Wasserabspaltung.

Oftmals bilden sich bei der Umsetzung der Bernsteinsäurederivate mit den entsprechenden Anilinen spontan die Halbanilide (worunter solche Verbindungen verstanden werden sollen, in denen bereits Amidbildung erfolgte, jedoch noch nicht der Ringschluß zum entsprechenden Imid). Diese Halbaniline können in einer weiteren günstigen Verfahrensvariante im Ein-Topf-Verfahren in Gegenwart von Kondensationsmitteln, wie z.B. Toluolsulfonsäure, zum entsprechenden Imid cyclisiert werden. Alternativ kann der Ringschluß, wie oben bereits beschrieben, durch Azeotropdestillation des Reaktionswassers bewirkt werden.

Oftmals werden auch spezielle Beispiele erfindungsgemäßer Verbindungen dadurch gewonnen, daß zunächst die entsprechenden 3-Halogen- bzw. Pseudohalogen-methyl-pyrrolidin-2.5-dione hergestellt werden und anschließend der an sich erwünschte Substituent R in an sich bekannter Weise durch nukleophile Substitution eingeführt wird.

Die 3-Halogen- bzw. Pseudohalogen-methyl-pyrrolidin-2.5-dione sind wiederum durch Addition von Halogenwasserstoff oder Pseudohalogenwasserstoff an die entsprechenden Imide der Itakonsäure zugänglich. Die 3-Brommethyl-pyrrolidin-2.5-dione sind in einer besonders bevorzugten Verfahrensweise durch Cyclisieren der entsprechenden Itakonsäureanilide in Gegenwart von Acetylbromid zugänglich, wobei gleichzeitig Ringschluß zum Imid und Bromwasserstoffaddition an der Methylengruppe der Itakonsäure erfolgt.

Die zur Herstellung der erfindungsgemäßen Verbindungen als Ausgangssubstanzen benötigten Bernsteinsäurederivate sind in an sich bekannter Weise durch entsprechende Umsetzungen mit Itakonsäure oder Itakonsäurederivaten zugänglich. Beispielsweise wird 2-Chlormethyl-bernsteinsäureanhydrid durch Umsetzen von Itakonsäure mit Thionylchlorid in polaren organischen Lösungsmitteln gewonnen. Analog wird 2-Brommethylbernsteinsäureanhydrid durch Behandeln mit Thionylbromid gewonnen. Ferner ist 2-Brommethylbernsteinsäure in an sich bekannter Weise durch Addition von Bromwasserstoffsäure an Itakonsäure darstellbar. Die sonstig benötigten substituierten Methylbernsteinsäuren sind aus den genannten Halogenmethylbernsteinsäuren durch nukleophile Substitution an der Halogenmethyl-Funktion zugänglich. Die Methansulfonylgruppe wird zweckmäßigerweise durch Oxidation der entsprechenden Methylthioverbindung gewonnen.

Die zur Herstellung der erfindungsgemäßen Verbindungen benötigten Aniline sind bekannte, zumeist im Handel erhältliche Verbindungen.

Die erfindungsgemäßen Verbindungen weisen fungitoxische Eigenschaften auf. Sie werden gegen Pilzbefall an Pflanzen bzw. an pflanzlichen Produkten eingesetzt.

Sie erweisen sich beispielsweise hochwirksam gegen alle Lebensformen von Botrytis cinerea und gegen deren Begleitpilze, wie Alternaria solani und Penicillium glaucum. Es ist insbesondere hervorzuheben, daß die erfindungsgemäßen Wirkstoffe auch gegen solche Botrytis-Stämme wirksam sind, die bereits gegen bekannte Dicarboxanilide Resistenzerscheinungen zeigen.

Ferner werden solche Pilze bekämpft wie Alternaria-Arten, Septoria-Arten, Verticillium dahliae, Colletotrichum-Arten, Monilia-Arten, Fusarium-Arten und Oomyceten, wie z.B. Pythium ultimum. Weiterhin sind die erfindungsgemäßen Wirkstoffe erfolgreich gegen phytopathogene Pilze, die dem Saatgut anhaften, einsetzbar, wie z.B. Tilletia tritici, Fusarium nivale und Helminthosporium-Arten.

Die erfindungsgemäßen Wirkstoffe zeichnen sich durch ein breites Wirkungsspektrum aus. So wird beispielsweise nicht nur die Botrytis bekämpft, sondern ebenso die typischen Begleitpilze. Mit den erfindungsgemäßen Wirkstoffen kann mithin der Gesamtkomplex der Pilzkrankheit behandelt werden und damit das bei einseitiger Behandlung oftmals beobachtete vermehrte Aufwachsen von Begleitpilzen verhindert werden.

Die Wirkstoffe eignen sich, ohne daß ihr Anwendungsgebiet darauf beschränkt wäre, z.B. zum Einsatz im Weinbau, im Gartenbau, insbesondere in Salatpflanzungen oder bei Zierpflanzen (Alpenveilchen, Geranien), in Zierrasen, beim Rapsanbau, beim Hopfenanbau, in Erdbeerpflanzungen und im Kernobstbau.

Die erfindungsgemäßen Pyrrolidin-2.5-dione sind ferner zur Konservierung geernteter Früchte verwendbar. Als weitere Anwendung wurd der Einsatz als Saatgutbeizmittel gefunden. Ferner sind die Wirkstoffe als technische Fungizide einsetzbar, z.B. in Holzschutzmitteln oder in Kunststoffmassen.

Die Applikation der Wirkstoffe erfolgt in an sich bekannter Weise auf den Lebensraum der Pilze durch beispielsweise Gießen, Verspritzen, Versprühen, Zerstäuben, Bestreichen, Behandeln des Saatguts (Beizen). Es kann dabei sowohl eine prophylaktische als auch eine kurative Wirkung erzielt werden.

Die erfindungsgemäßen Wirkstoffe können allein oder im Gemisch mit sonstigen Pestiziden, insbesondere fungiziden Mitteln ausgebracht werden. Im allgemeinen werden sie als Mischungen mit festen oder flüssigen Verdünnungsmitteln oder als Lösungen in festen oder flüssigen Lösungsmitteln verwendet, mit Wirkstoffgehalten von 0,005 bis 95 Gew.-%.

Die Mischungen bzw. Lösungen werden im allgemeinen als Emulsionskonzentrate, Pasten, Spritzpulver, Granulate oder Mikrokapseln hergestellt.

Emulsionskonzentrate und Pasten enthalten im allgemeinen 10 bis 60 Gew.-%, vorzugsweise 15 bis 50 Gew.-% Wirkstoff, 2 bis 25 Gew.-% Dispergierhilfsstoffe und organische Lösungsmittel und/oder Wasser.

Spritzpulver enthalten meistens 10 bis 80 Gew.-%, vorzugsweise 15 bis 70 Gew.-% Wirkstoff, 1 bis 10 Gew.-% Dispergierhilfsstoffe und 10 bis 89 Gew.-% inerte Bestandteile.

Granulate und Stäubemittel enthalten neben inerten Bestandteilen, Bindemitteln und/oder Überzugsstoffen 1 bis 10 Gew.-%, vorzugsweise 5 bis 10 Gew.-% Wirkstoff.

Erfindungsgemäß angewandt werden:

Als Dispergierhilfsstoffe z.B. Alkyl- und Arylsulfonate, Methylzellulose, polymere Sulfonsäuren und deren Salze, Polyalkohole, Fettsäureester, Fettalkoholether, Fettamine;

als organische Lösungsmittel z.B. Alkohole, wie Ethanol, Butanole, Dimethylformamid, Dimethylsulfoxid, N-Methylpyrrolidon, Aromaten, wie Toluol und Xylole;

als inerte Bestandteile z.B. Kaolin, China-Clay, Talkum, Calciumcarbonat, hochdisperse Kieselsäure, Kieselgele, Kieselgur, Diatomenerde, Bims, Maisschrot, Cyclodextrine, Verdickungsmittel, wie Stärke und Carboxymethylzellulose;

als Bindemittel z.B. Magnesiumsulfat, Gips, Gummiarabikum.

Beispielsweise werden die erfindungsgemäßen Fungizide wie folgt formuliert:

1. Emulsionskonzentrat:

20 Gew.-% Wirkstoff
10 Gew.-% handelsübliches epoxidiertes Anhydrosorbitmonolaurat (Handelsname "Tween-Twenty")
70 Gew.-% Dimethylformamid

2. Spritzpulver:

20 Gew.-% Wirkstoff
5 Gew.-% Ammoniumligninsulfonat (Handelsname "Totanin")
10 Gew.-% Natriumoleylmethyltaurid (Handelsname "Arcopon T Konz")
65 Gew.-% Kaolin

Die Erfindung wird nun anhand von Beispielen näher erläutert:

Beispiel 1

Herstellung von 3-Chlormethyl-1-(4-fluorphenyl)-pyrrolidin-2.5-dion

a) Einer Lösung von 130 g (1 Mol) Itakonsäure in 220 ml Ethylacetat wurden 126 g (1,06 Mol) Thionylchlorid zugefügt. Das Gemisch wurde 5 Tage bei Raumtemperatur aufbewahrt. Danach wurde bis zum Ende der Gasentwicklung ($SO_2$, HCl) auf 80 °C erwärmt. Schließlich wurde destillativ aufgearbeitet. Es wurden 140 g, entsprechend 92 % d.Th. an 2-Chlormethyl-bernsteinsäureanhydrid erhalten.

   Siedebereich bei 0,25 mbar, 118 bis 124 °C

b) In eine Lösung von 33,3 g (0,3 Mol) 4-Fluoranilin und 4-Toluolsulfonsäure in Xylol wurde bei Raumtemperatur unter Rühren eine xylolische Lösung von 44,6 (0,3 Mol) 2-Chlormethyl-bernsteinsäureanhydrid zugetropft. Die Temperatur des Reaktionsgemisches stieg dabei auf 40 °C an. Es entstand eine Suspension des schwer löslichen Halbanilids. Nach weiterem 30-minütigem Rühren wurde auf Rückfluß erwärmt und das sich bildende Reaktionswasser azeotrop abdestilliert und über einen Wasserabscheider entfernt. Nachdem 5 ml Wasser abgetrennt waren, wurde abgekühlt, wobei das Zielprodukt in kristalliner Form anfiel. Das Produkt wurde noch aus Toluol umkristallisiert. Die Ausbeute betrug 42 % d.Th.

Schmelzpunkt 135 °C

c) In einer Verfahrensvariante zu b) wurde zu einer toluolischen Lösung von 33,3 (0,3 Mol) 4-Fluoranilin unter Rühren bei Raumtemperatur eine toluolische Lösung von 44,6 g (0,3 Mol) 2-Chlormethyl-bernsteinsäureanhydrid getropft. Die Temperatur des Reaktionsgemisches stieg dabei auf 40 °C. Zu der erhaltenen Suspension des Halbanilids wurden unter Rühren bei 60 °C 0,35 Mol Acetylchlorid so langsam zugetropft, daß die auftretende Gasentwicklung gut beherrschbar war. Nach 3 Stunden wurde auf 100 °C erhitzt und das Reaktionsgemisch so lange auf dieser Temperatur gehalten, bis die Gasentwicklung beendet war. Das Halbanilid löste sich dabei auf. Beim Abkühlen des Reaktionsgemisches kristallisierte das Zielprodukt aus. Die Ausbeute betrug 93 % d.Th.

Beispiel 2

Herstellung von 3-Brommethyl-1-(4-chlorphenyl)-pyrrolidin-2.5-dion

a) In eine toluolische Lösung von 33,6 g (0,3 Mol) Itakonsäureanhydrid wurde unter Rühren eine toluolische Lösung von 50,1 g (0,3 Mol) 4-Chloranilin zugetropft. Die Reaktionstemperatur des Reaktionsgemisches betrug 40 °C. Es fiel eine Suspension des entsprechenden Halbanilids an, der nun unter Rühren bei 60 °C 28,1 g (0,36 Mol) Acetylchlorid so langsam zugetropft wurde, daß eine mäßige Gasentwicklung entstand. Nach 3 Stunden wurde auf 100 °C erhitzt und das Reaktionsgemisch so lange bei dieser

Temperatur belassen, bis die Gasentwicklung beendet war. Nach dem Abkühlen des Reaktionsgemisches kristallisierte 1-(4-Chlorphenyl)-3-methylen-pyrrolidin-2.5-dion aus. Die Ausbeute betrug 93 % d.Th.

b) In eine Lösung von 41,8 g (0,25 Mol) des Produkts gemäß a) in Chloroform wurde gasförmiger Bromwasserstoff eingeleitet, bis die Gewichtskontrolle einen 15 Gew.-%-igen Überschuß an HBr anzeigte. Das Gemisch wurde noch 3 Stunden bei Zimmertemperatur gerührt. Nach dem Einengen der Lösung kristallisierte das Zielprodukt aus. Die Ausbeute betrug 95 % d.Th.

Schmelzpunkt 134 °C

c) In einer weiteren Verfahrensvariante wurde das Itakonsäure-Halbanilid gemäß a) abfiltriert, mit Petrolether gewaschen und getrocknet. 46,3 g (0,25 Mol) des Halbanilids wurden in Essigsäureethylester suspendiert. Der Suspension wurden 33,8 g (0,27 Mol) Acetylbromid langsam zugetropft. Danach wurde 4 Stunden am Rückfluß gekocht, das Lösungsmittel abgedampft und der Rückstand aus Isopropanol/Methanol umkristallisiert. Das Zielprodukt wurde in einer Ausbeute von 72 % d.Th. erhalten.

## Beispiel 3

Herstellung von 3-Jodmethyl-1-(4-fluorphenyl)-pyrrolidin-2.5-dion

Eine Lösung von 15,2 g (0,06 Mol) 3-Chlormethyl-1-(4-fluorphenyl)-pyrrolidin-2.5-dion (gemäß Beispiel 1) und 10,7 g (0,066 Mol) Natriumjodid in Aceton wurde 8 Stunden am Rückfluß gekocht. Danach wurde das ausgefallene Natriumchlorid abfiltriert und das Filtrat eingeengt. Danach wurde mit Chloroform extrahiert und der Extrakt erneut eingeengt. Dabei fiel das Zielprodukt in einer 92 %-igen Ausbeute an.

Schmelzpunkt 156 °C.

Beispiel 4

Herstellung von 3-Cyanomethyl-1-(4-fluorphenyl)-pyrrolidin-2.5-dion

Es wurde ein Gemisch aus 48,3 g (0,2 Mol) 3-Chlormethyl-1-(4-fluorphenyl)-pyrrolidin-2.5-dion (gemäß Beispiel 1), 300 ml Methylenchlorid und 7 g Hexadecyltrimethylammoniumchlorid, gelöst in 80 ml Wasser, hergestellt. Dem Gemisch wurde bei 0 °C eine Lösung von 28,6 g (0,43 Mol) Kaliumcyanid in 90 ml Wasser zugetropft. Das Gemisch wurde noch 4 Stunden bei 0 °C und danach 12 Stunden bei 20 °C gerührt. Danach wurden die Phasen getrennt, die organische Phase mit Wasser gewaschen und mit Natriumsulfat getrocknet. Schließlich wurde Methylenchlorid abgezogen und der Rückstand in 250 ml Toluol aufgenommen. Beim Stehenlassen kristallisierte das Zielprodukt aus. Die Ausbeute betrug 44,1 %.

Schmelzpunkt 97 °C.

Beispiel 5

Herstellung von 3-Thiocyanatomethyl-1-(4-fluorphenyl)-pyrrolidin-2.5-dion

Es wurde die Arbeitsweise gemäß Beispiel 4 wiederholt, mit der Abänderung, daß anstatt 0,43 Mol Kaliumcyanid 0,43 Mol Kaliumthiocyanat eingesetzt wurden.

Das Zielprodukt fiel in einer Ausbeute von 52 % d.Th. an.

Schmelzpunkt 88 °C.

Beispiel 6

Herstellung von 1-(4-Fluorphenyl)-3-methansulfonylmethyl-2.5-pyrrolidindion

a) Einer Lösung von 48,3 g (0,2 Mol) 3-Chlormethyl-1-(4-fluorphenyl)-2.5-pyrrolidindion in 130 ml Aceton wurde

bei 0 °C eine ethanolische Lösung von 0,2 Mol Natriummethylmercaptid zugetropft. Nach weiterem 4-stündigem Rühren bei 20 °C wurde das gebildete Salz abfiltriert, das Filtrat eingeengt und der Rückstand aus Methanol umkristallisiert. Es wurde in einer Ausbeute von 70,3 % d.Th. 1-(4-Fluorphenyl)-3-methylthiomethyl-2.5-pyrrolidindion erhalten.

b) 12,5 g (0,05 Mol) des Produkts gemäß a) wurden in 30 ml Ethylacetat gelöst und nach Zugabe von 0,5 $cm^3$ einer 0,1 %-igen Ammoniummolybdat-Lösung, 0,36 ml konzentrierter Essigsäure und 12 ml 10 %-iger, wäßriger Natriumacetat-Lösung mit 5 ml 60 %-igem Perhydrol unter Rühren bei 50 °C oxidiert. Zur Vervollständigung der Reaktion wurde noch auf 80 °C unter Rühren erwärmt. Schließlich wurde das Zielprodukt mit zweimal 100 ml Ethylacetat aus dem Gemisch extrahiert. Danach wurde eingeengt, wobei das Zielprodukt als kristalliner Niederschlag anfiel. Die Ausbeute betrug 63,7 % d.Th.

Schmelzpunkt 162 bis 163 °C.

Im folgenden werden Wirkstoffe gemäß Stand der Technik in tabellarischer Form aufgeführt, die in den folgenden Beispielen mit den entsprechenden Kennbuchstaben bezeichnet sind:

A 3-Brom-3-brommethyl-1-(4-fluorphenyl)-2.5-pyrrolidindion (entsprechend Jap. Kokai 77/38.021)
B 3-Chlor-1-(3.5-dichlorphenyl)-2.5-pyrrolidindion (entsprechend GB-PS 14 62 140)
C 3-Chlor-1-(2.6-diethylphenyl)-2.5-pyrrolidindion (entsprechend Jap. Kokai 75/125.743)
D 3.4-Dichlor-1-(4-fluorphenyl)-1-H-pyrrol-2.5-dion (entsprechend Jap. Kokai 71/2681)
E 3-Chlormethyl-1-(4-fluorphenyl)-1-H-pyrrol-2.5-dion (entsprechend Jap. Kokai 75/107.137)

F 1-(4-Chlorphenyl)-3-methylen-2.5-pyrrolidindion (entsprechend DE-OS 11 53 205)

Im folgenden werden die erfindungsgemäßen Wirkstoffe mit Kennziffern bezeichnet, die aus der vorstehenden Aufstellung erfindungsgemäßer Wirkstoffe zu entnehmen sind.

## Beispiel 7

Lichtbeständigkeitstest

Es wurde jeweils 1 mg an Substanz, gelöst in einem flüchtigen Lösungsmittel, in eine Abdampfschale gegeben und anschließend das Lösungsmittel verdampft. Die so erhaltene dünne Schicht der auf Lichtbeständigkeit zu prüfenden Substanz wurde anschließend mit einer Bestrahlungslampe beleuchtet, deren Strahlungsbereich dem natürlichen Sonnenlicht entsprach (Xenonstrahler mit Filtersystem; Wellenlängenbereich 300 bis 830 nm). Es wurden jeweils die Halbwertszeiten der Prüfsubstanzen unter konstanten Bestrahlungsbedingungen ermittelt. Die Analyse erfolgte mittels Hochdruckflüssigkeitschromatographie.

Tabelle 1

| Wirkstoff | Halbwertszeit in min |
|---|---|
| 2 | 240 |
| 4 | 890 |
| 17 | 310 |
| 83 | 740 |
| E | 23 |

Beispiel 8

Sporenkeimtest

50 µl einer Lösung oder Suspension eines Wirkstoffs mit einem Gehalt von 250 bzw. 16 ppm Aktivsubstanz wurden zusammen mit 50 µl einer Sporensuspension, hergestellt durch Abschwemmen der Sporen von einer Agarkultur mit einer Nährlösung, die pro Liter 10 g Zucker, 1 g Glykol, 1 g $KH_2PO_4$ und 0,5 g $MgSO_4$ enthielt, in den Hohlschliff von Hohlschliffobjektträgern eingebracht. Die Objektträger wurden bei 20 °C 48 Stunden in einer Petrischale, deren Boden mit angefeuchtetem Filterpapier bedeckt war, aufbewahrt.

Danach wurde das Verhältnis der gekeimten und der nicht gekeimten Sporen gegen eine unbehandelte Kontrollprobe verglichen.

Der Wirkungsgrad wird in % nach der folgenden Formel angegeben:

$$100 - \frac{\text{Anzahl der gekeimten Sporen, behandelt}}{\text{Anzahl der gekeimten Sporen, unbehandelt}} \times 100$$

Die Ergebnisse sind in den folgenden Tabelle 2 und 3 zusammengestellt.

Tabelle Nr. 2: Fungitoxizität erfindungsgemäßer 2,5-Pyrrolidindione bei 250 ppm Wirkstoffkonzentration in %

| Wirkstoff | Alternaria solani | Botrytis cinerea | Fusarium culmorum | Fusarium nivale | Colletotrichium coffeanum | Verticillium dahliae | Penicillium glaucum |
|---|---|---|---|---|---|---|---|
| 1 | 100 | 70 | 70 | 80 | 80 | 100 | 50 |
| 2 | 100 | 100 | 100 | 100 | 100 | 100 | 100 |
| 3 | 80 | 100 | 80 | 100 | 100 | 100 | 80 |
| 4 | 100 | 80 | 100 | 100 | 100 | 100 | 80 |
| 6 | 80 | 70 | 100 | 100 | 100 | 100 | 80 |
| 7 | 70 | 70 | 100 | 100 | 100 | 100 | 80 |
| 8 | 100 | 100 | 100 | 100 | 100 | 100 | 100 |
| 9 | 100 | 100 | 80 | 100 | 100 | 100 | 100 |
| 10 | 100 | 100 | 100 | 100 | 100 | 100 | 100 |
| 11 | 100 | 100 | 100 | 100 | 100 | 100 | 100 |
| 12 | 100 | 100 | 100 | 100 | 100 | 100 | 100 |
| 13 | 100 | 100 | 100 | 100 | 100 | 100 | 100 |
| 14 | 100 | 100 | 100 | 100 | 100 | 100 | 100 |
| 16 | 80 | 50 | 70 | 100 | 100 | 100 | 60 |
| 19 | 100 | 100 | 60 | 100 | 100 | 100 | 80 |
| 20 | 80 | 100 | 80 | 100 | 100 | 100 | 100 |
| 38 | 100 | 100 | 80 | 100 | 100 | 100 | 80 |
| 40 | 100 | 70 | 70 | 100 | 100 | 100 | 80 |
| 41 | 100 | 60 | 100 | 100 | 100 | 100 | 100 |

Tabelle Nr. 2:
(Fortsetzung)

| Wirkstoff | Alternaria solani | Botrytis cinerea | Busarium culmorum | Fusarium nivale | Colletotrichium coffeanum | Verticillium dahliae | Penicillium glaucum |
|---|---|---|---|---|---|---|---|
| 42 | 100 | 100 | 100 | 100 | 80 | 100 | 80 |
| 46 | 90 | 100 | 80 | 100 | 100 | 100 | 100 |
| 47 | 100 | 100 | 80 | 100 | 100 | 100 | 80 |
| 48 | 80 | 60 | 20 | 100 | 100 | 80 | 70 |
| 49 | 80 | 90 | 100 | 100 | 100 | 100 | 100 |
| 73 | 90 | 100 | 90 | 100 | 100 | 100 | 100 |
| 74 | 100 | 100 | 100 | 100 | 100 | 100 | 100 |
| 82 | 100 | 100 | 100 | 100 | 90 | 100 | 80 |
| 83 | 100 | 100 | 100 | 100 | 100 | 100 | 100 |
| 84 | 100 | 100 | 100 | 100 | 100 | 100 | 100 |
| 85 | 100 | 100 | 100 | 100 | 100 | 100 | 100 |
| 86 | 100 | 100 | 100 | 100 | 100 | 100 | 80 |
| 87 | 100 | 100 | 100 | 100 | 100 | 100 | 100 |
| 88 | 100 | 100 | 100 | 100 | 100 | 100 | 100 |
| 90 | 70 | 100 | 100 | 100 | 100 | 100 | 100 |
| 91 | 100 | 100 | 100 | 100 | 100 | 100 | 100 |
| 92 | 100 | 100 | 100 | 100 | 100 | 100 | 100 |
| 94 | 90 | 100 | 90 | 100 | 100 | 100 | 100 |
| 100 | 80 | 70 | 60 | 100 | 100 | 100 | 70 |
| 123 | 100 | 100 | 100 | 100 | 100 | 100 | 100 |
| 135 | 80 | 60 | 60 | 100 | 100 | 100 | 80 |

Tabelle Nr. 3: Fungitoxizität erfindungsgemäßer 2,5-Pyrrolidindione und von Vergleichssubstanzen bei 16 ppm Wirkstoffkonzentration

| Wirkstoff | Alternaria solani | Botrytis cinerea | Fusarium culmorum | Fusarium nivale | Colletotrichium coffeanum | Verticillium dahliae | Penicillium glaucum |
|---|---|---|---|---|---|---|---|
| 4 | 100 | 60 | 70 | 100 | 100 | 40 | 60 |
| 7 | 50 | 50 | 70 | 100 | 100 | 50 | 50 |
| 8 | 80 | 80 | 80 | 100 | 80 | 100 | 80 |
| 10 | 80 | 50 | 0 | 100 | 80 | 60 | 80 |
| 13 | 100 | 80 | 40 | 80 | 100 | 80 | 90 |
| 38 | 60 | 70 | 40 | 80 | 100 | 80 | 40 |
| 47 | 100 | 100 | 60 | 40 | 100 | 50 | 40 |
| 74 | 40 | 80 | 40 | 100 | 100 | 100 | 60 |
| 84 | 100 | 60 | 60 | 100 | 100 | 60 | 40 |
| 87 | 100 | 60 | 0 | 100 | 60 | 80 | 100 |
| A | 0 | 0 | 0 | 0 | 10 | 10 | 0 |
| B | 60 | 40 | 0 | 80 | 80 | 60 | 60 |
| C | 40 | 60 | 60 | 80 | 80 | 60 | 40 |
| D | 50 | 70 | 30 | 30 | 90 | 90 | 30 |

Beim Vergleich der in Tabelle 3 aufgeführten Ergebnisse wird deutlich, daß die erfindungsgemäßen 2.5-Pyrrolidindione eine gegenüber den Vergleichssubstanzen erhöhte fungitoxische Wirkung und insbesondere ein wesentlich verbreitertes Wirkungsspektrum aufweisen. Die Vergleichssubstanzen weisen jeweils gleiche oder ähnliche Strukturelemente auf. Daraus ist ersichtlich, daß die erhöhte fungizide Wirksamkeit der erfindungsgemäßen Substanzen auf die erfindungsgemäße Auswahl der Substituenten am Bernsteinsäureimid-Gerüst zurückgeführt werden muß.

Beispiel 9

Saatgutbeizmitteltest - Wirksamkeit gegen Helminthosporium gramineum

Natürlich infiziertes Gerstensaatgut wurde mit den in der folgenden Tabelle aufgeführten Wirksubstanzen gebeizt und anschließend in Schalen mit Torf/Sand-Gemisch ausgelegt, wobei die Saatkörner mit einer 2,5 cm dicken Gemischschicht abgedeckt waren. Zunächst wurden die verschlossenen Schalen 2 Wochen lang im abgedunkelten Klimaschrank bei 3 bis 6 °C aufgestellt. Danach wurden die Deckel entfernt und die Schalen weitere 2 Wochen bei Licht und einer Temperatur von 20 °C unter Gewächshausbedingungen aufbewahrt. Die herangewachsenen Pflanzen wurden auf Schädigungen untersucht. Es wurde die Anzahl kranker bzw. geschädigter Pflanzen ermittelt und mit unbehandelten Kontrollen verglichen. Die Ergebnisse sind in der folgenden Tabelle zusammengestellt:

Tabelle Nr. 4: Wirksamkeit in % erfindungsgemäßer Substanzen und Vergleichsverbindungen bei 500 ppm Wirkstoffkonzentration gegen Helminthosporium gramineum

| Stoffnummer | %-Wirksamkeit |
|---|---|
| 3 | 70 |
| 4 | 90 |
| 14 | 80 |
| 15 | 80 |
| 19 | 70 |
| 39 | 70 |
| 40 | 70 |
| 42 | 70 |
| 74 | 90 |
| 82 | 90 |
| 83 | 90 |
| 84 | 90 |
| 85 | 80 |
| 86 | 100 |
| 89 | 70 |
| 92 | 80 |
| 94 | 80 |
| 62 | 100 |
| A | 40 |
| B | 35 |
| C | 15 |
| D | 50 |
| E | 15 |

Beispiel 10

Traubensafttest

20 ml einer Nährlösung aus Traubensaft und destilliertem Wasser im Verhältnis 1 : 1 wurden in Petrischalen eingefüllt und mit den in der folgenden Tabelle angeführten Wirkstoffen versetzt. Die Wirkstoffkonzentration betrug 31 ppm. Anschließend wurden die Versuchsansätze mit jeweils 50 µl einer Botrytis-Sporensuspension, hergestellt durch Abschwemmen der Botrytis-Sporen von einer Agarkultur mit destilliertem Wasser, beimpft.

Nach einer Bebrütungsdauer von 10 bzw. 20 Tagen bei 20 °C wurde das Ausmaß der Pilzentwicklung auf der Nährlösungsoberfläche beurteilt.

Der Wirkungsgrad wurde in % nach der folgenden Formel errechnet:

$$100 - \frac{\text{Pilzwachstum, behandelt}}{\text{Pilzwachstum, unbehandelt}} \times 100$$

Tabelle Nr. 5: Wirksamkeit erfindungsgemäßer 2,5-Pyrrolidindione und Vergleichsmittel in %-Graden bei 31 ppm Wirkstoffkonzentration nach 10 bzw. 20 Tagen Einwirkungsdauer

| Wirkstoff Nr. | %-Wirksamkeit nach 10 Tagen | nach 20 Tagen |
|---|---|---|
| 2 | 100 | 90 |
| 3 | 100 | 100 |
| 12 | 100 | 90 |
| 39 | 100 | 80 |
| 74 | 100 | 80 |
| 83 | 100 | 100 |
| 84 | 100 | 100 |
| 85 | 100 | 90 |
| 86 | 100 | 100 |
| 87 | 100 | 100 |
| 88 | 100 | 100 |
| 90 | 100 | 100 |
| 92 | 100 | 100 |
| 94 | 100 | 100 |
| 123 | 100 | 80 |
| B | 40 | 10 |
| D | 80 | 60 |

Beispiel 11

Traubensafttest mit resistenten Botrytis cinerea-Stämmen

Es wurde die Arbeitsweise gemäß Beispiel 10 wiederholt, mit der Abänderung, daß mit Botrytis-Sporen von Botrytis cinerea-Stämmen beimpft wurde, die gegen N-(3.5-Dichlorphenyl)-imide Resistenzerscheinungen zeigen. Die Wirkstoffkonzentration betrug 62 ppm.

Als Vergleichsmittel wurde der Wirkstoff "Ronilan" (3-(3.5-Dichlorphenyl)-5-ethenyl-5-methyl-2.4-oxazolidindion) verwendet. Die Bebrütungsdauer betrug 20 Tage.

Die Ergebnisse sind in der folgenden Tabelle zusammengestellt.

Tabelle 6: Wirksamkeit erfindungsgemäßer 2.5-Pyrrolidindione sowie des Vergleichsmittels in %

| Wirkstoff | sensibler Stamm | resistenter Stamm A | resistenter Stamm B |
|---|---|---|---|
| 73 | 100 | 100 | 100 |
| 74 | 100 | 100 | 100 |
| 83 | 100 | 100 | 100 |
| 90 | 100 | 100 | 100 |
| Ronilan * | 90 | 30 | 30 |

* = Eingetragenes Warenzeichen der BASF AG

Beispiel 12

Botrytis-Test an lebenden Bohnenpflanzen

Junge Bohnenpflanzen, die bereits die ersten dreiblättrigen Triebe ausgebildet hatten, wurden mit einer Spritzbrühe, die 500 ppm Wirkstoffkonzentration aufwies, tropfnaß gespritzt. Nach Antrocknung des Spritzbelages wurden die Bohnenpflanzen durch Besprühen mit einer Botrytis cinerea-Sporensuspension künstlich infiziert. Die so behandelten Pflanzen wurden bei 20 °C und 95 %-iger Luftfeuchtigkeit aufbewahrt und nach 6 Tagen auf Befall bonitiert. Unbehandelte Pflanzen dienten als Kontrolle.

Tabelle Nr. 7: Wirksamkeit in % von erfindungsgemäßen 2,5-Pyrrolidindionen und von Vergleichssubstanzen gegen Botrytis cinerea an Bohnenpflanzen bei je 500 ppm Wirkstoffkonzentration

| Wirkstoff | Wirksamkeit in % |
|---|---|
| 1 | 90 |
| 2 | 90 |
| 4 | 70 |
| 5 | 75 |
| 6 | 75 |
| 7 | 85 |
| 8 | 80 |
| 12 | 80 |
| 38 | 70 |
| 46 | 80 |
| 47 | 70 |
| 73 | 70 |
| 74 | 80 |
| 82 | 70 |
| 83 | 80 |
| 84 | 70 |
| 85 | 85 |
| 87 | 80 |
| 90 | 95 |
| 91 | 70 |
| 92 | 75 |
| 94 | 80 |
| 123 | 70 |
| A | 60 |
| B | 60 |
| D | 30 |

## Beispiel 13

Botrytis-Test an lebenden Bohnenpflanzen mit resistenten Botrytis cinerea-Stämmen

Es wurde die Arbeitsweise gemäß Beispiel 12 wiederholt, mit der Abänderung, daß mit Botrytis cinerea-Stämmen infiziert wurde, die Resistenzerscheinungen gegenüber N-(3.5-dichlorphenyl)-imiden zeigten.

Als Vergleichssubstanzen gemäß Stand der Technik dienten die Wirkstoffe "Ronilan" und "Rovral" (3-(3.5-Dichlorphenyl)-N-(1-methylethyl)-2.4-dioxo-1-imidazolidincarboxamid)

Tabelle 8: Fungitoxische Wirksamkeit in % erfindungsgemäßer 2.5-Pyrrolidindione und von Vergleichsmitteln gegen resistente Botrytis-Stämme bei je 500 ppm Wirkstoffkonzentration

| Wirkstoff | sensibler Stamm | resistenter Stamm A | resistenter Stamm B |
|---|---|---|---|
| 2 | 90 | 85 | 90 |
| 73 | 70 | 80 | 70 |
| 74 | 80 | 80 | 70 |
| 83 | 80 | 80 | 75 |
| 90 | 90 | 80 | 90 |
| Ronilan * | 85 | 25 | 45 |
| Rovral ** | 87 | 20 | 40 |

* = Eingetragenes Warenzeichen der BASF AG
**= Eingetragenes Warenzeichen der Rhône-Poulenc SA

## Beispiel 14

Wirksamkeit gegen Pythium ultimum bei Bodenapplikation

Die in der folgenden Tabelle aufgeführten Wirkstoffe (Wirkstoffkonzentration 500 ppm) wurden gleichmäßig in Erde gemischt, die mit Pythium ultimum künstlich infiziert war. Die so behandelte Erde wurde in Plastiktöpfe gefüllt (je 4 Wiederholungen pro Testsubstanz) und mit je 10 Erbsensamen besät. Diese Töpfe wurden 10 Tage bei 24 bis 26 °C

und einer Luftfeuchte von 75 bis 90 % aufbewahrt. Danach wurde die Zahl der gesunden, aufgelaufenen Pflanzen bestimmt. Der Wirkungsgrad wurde durch Vergleich mit infizierten, aber unbehandelten Erdproben errechnet.

Die Ergebnisse sind in der folgenden Tabelle zusammengestellt:

Tabelle Nr. 9: Wirksamkeit in % von erfindungsgemäßen 2.5-Pyrrolidindionen und von Vergleichsmitteln gegen Pythium ultimum bei 500 ppm Wirkstoffkonzentration

| Wirkstoff | % Wirksamkeit |
|---|---|
| 3 | 80 |
| 4 | 100 |
| 5 | 100 |
| 8 | 100 |
| 9 | 100 |
| 15 | 100 |
| 17 | 80 |
| 19 | 100 |
| 20 | 85 |
| 40 | 100 |
| 46 | 100 |
| 73 | 100 |
| 74 | 100 |
| 85 | 100 |
| 86 | 100 |
| 123 | 100 |
| A | 40 |
| B | 0 |
| D | 0 |
| E | 50 |

Patentansprüche:

1. Verbindungen der allgemeinen Formel

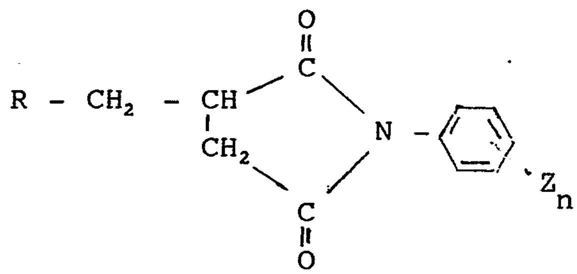

wobei

R F, Cl, Br, J, CN, SCN und Methansulfonyl bedeutet,

Z für F, Cl, Br, J, $NO_2$, CN, SCN, Sulfamoyl, Phenoxy, Alkyl mit 1 bis 3 Kohlenstoffatomen, Halogenalkyl mit 1 bis 3 Kohlenstoffatomen und 1 bis 7 Halogenatomen aus der Gruppe F, Cl, Br; Alkoxy mit 1 bis 3 Kohlenstoffatomen, Halogenalkoxy mit 1-3 Kohlenstoff- und 1-4 Halogenatomen aus der Gruppe F, Cl, Br; Allyloxy und Ethoxycarbonyl steht,

n 0, oder, mit der Maßgabe, daß n 1, 2, 3 oder 4 ist, für folgenden Substitutionstyp steht: 2-, 4-; 2.3-, 2.4-, 2.5-, 2.6-, 3.4-; 2.3.4-, 2.3.5-, 2.3.6-, 2.4.6-, 3.4.5-; 2.3.4.5-, 2.4.5.6-, 2.3.5.6-;

2. Fungizid wirksame Zusammensetzungen, die mindestens eine Verbindung nach Anspruch 1 enthalten.

3. Verfahrens zur Herstellung von Verbindungen nach Anspruch 1, dadurch gekennzeichnet, daß Bernsteinsäuren, deren Säurechloride bzw. deren Anhydride, die in der 2-Stellung durch einen

$R - CH_2$-Rest

substituiert sind, mit Anilinen der Formel

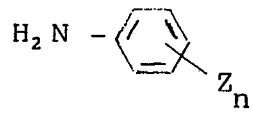

wobei

R, Z und n die gemäß Anspruch 1 gegebene Bedeutung besitzen,

umgesetzt werden.

4. Verwendung der Verbindungen nach Anspruch 1 als Fungizide.

Europäisches Patentamt

**EUROPÄISCHER RECHERCHENBERICHT**

**0 173 284**

EP 85110695.5

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| A | CHEMICAL ABSTRACTS, Band 87, 15. August 1977, Columbus, Ohio, USA<br>KONAGAI, YOSHIHIRO et al. "Phenylsuccinimides as fungicides"<br>Seite 187, Spalte 2, Zusammenfassung-Nr. 64 058d | 1-4 | C 07 D 207/40<br>A 01 N 43/36 |
| D,A | & Japan. Kokai 77 38,021<br>-- | | |
| A | CHEMICAL ABSTRACTS, Band 84, 19. Jänner 1976, Columbus, Ohio, USA<br>SHIGEMATSU, TAICHIRO et al. "N-Phenyl-chloromethylmaleimides as fungicides"<br>Seite 133, Spalte 1, Zusammenfassung-Nr. 13 496a | 1-4 | |
| D,A | & Japan. Kokai 75 107,137<br>-- | | RECHERCHIERTE SACHGEBIETE (Int. Cl.4) |
| A | DE - A1 - 3 204 953 (CONSORTIUM FÜR ELEKTROCHEMISCHE INDUSTRIE GMBH)<br>* Patentansprüche 1,3,4 *<br>-- | 1-4 | C 07 D 207/00<br>A 01 N |
| D,A | PESTICIDE SCIENCE, Band 12, 1981<br>M. GRINDLE "Variations Among Field Isolates of Botrytis Cinerea in Their Sensitivity to Antifungal Compounds"<br>Seiten 305-312<br>* Seite 305 *<br>---- | 1,2,4 | |

Der vorliegende Recherchenbericht wurde für alle Patentansprüche erstellt.

| Recherchenort | Abschlußdatum der Recherche | Prüfer |
|---|---|---|
| WIEN | 29-10-1985 | HEIN |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82